# EUROPEAN PATENT SPECIFICATION

(11) **EP 2 604 248 B1**
(45) Date of publication and mention of the grant of the patent: **25.10.2017**
(21) Application number: 11193275.2
(22) Date of filing: 13.12.2011
(51) Int. Cl.: A61K 8/04, A61K 8/365, A61Q 15/00, A61K 8/02, A61K 8/06

(54) **Oil-in-water cosmetic composition**
Öl-in-Wasser-Zusammensetzung
Composition cosmétique huile dans l'eau

(43) Date of publication of application: 19.06.2013
(73) Proprietor: Unilever PLC, London EC4Y 0DY (GB); Unilever N.V., 3013 AL Rotterdam (NL)
(72) Inventor: Graham, Peter, Wirral, Merseyside CH 63 3JW (GB); Kowalski, Adam Jan, Wirral, Merseyside CH 63 3JW (GB); Marriott, Robert Edward, Wirral, Merseyside CH 63 3JW (GB)
(74) Representative: Whaley, Christopher

(56) References cited:
- EP-A1- 2 269 565
- EP-A2- 1 151 743
- WO-A1-00/64302
- WO-A2-2010/031657
- US-B1- 6 171 581
- KIRILOV P ET AL: "A new type of colloidal dispersions based on nanoparticles of gelled oil", COLLOIDS AND SURFACES. A, PHYSICACHEMICAL AND ENGINEERING ASPECTS, ELSEVIER, AMSTERDAM, NL, vol. 328, no. 1-3, 1 October 2008 (2008-10-01), pages 1-7, XP024340258, ISSN: 0927-7757, DOI: 10.1016/J.COLSURFA.2008.06.011 [retrieved on 2008-06-13]
- PLAMEN KIRILOV ET AL: "Rheological Characterization of a New Type of Colloidal Dispersion Based on Nanoparticles of Gelled Oil", THE JOURNAL OF PHYSICAL CHEMISTRY B, vol. 113, no. 32, 13 August 2009 (2009-08-13), pages 11101-11108, XP55028161, ISSN: 1520-6106, DOI: 10.1021/jp905260s

## Description

The present invention relates to oil-in-water emulsion cosmetic compositions and to methods of making the same. It particularly relates to aqueous antiperspirant compositions suitable for roll-on application and to aqueous cosmetic compositions comprising 12-hydroxystearic acid.

Cosmetic compositions in the form of oil-in-water emulsions have been known for some time. They have found use in many types of cosmetics, notably skin creams and lotions. They have also been used as the basis for antiperspirant compositions, in particular those designed for application from a roll-on dispenser.

Roll-on application is achieved using a roll-on dispenser, in which a roller, most usually a spherical ball, partly protrudes outside a housing at the mouth of the dispenser that enables the roller to rotate and transport fluid from within the dispenser by adherence to its surface. Users apply the composition by rolling the roller across the skin surface.

Typically, oil-in-water emulsion cosmetic compositions suitable for roll-on application are emulsions with relatively small amounts of oil emulsified in an aqueous continuous phase. It is much less common for such compositions to comprise solid components as these can lead to problems in dispensing *via* the roll ball and possible formulation and/or stability difficulties.

Stability difficulties may manifest themselves during storage and/or transportation. It will be realised that such storage and/or transportation may need to take place over a wide range of temperature, from sub-zero up to 45°C and above in some locations.

Many antiperspirant compositions comprising 12-hydroxystearic acid are disclosed in the prior art because 12-hydroxystearic acid has been widely used as a structurant or thickener in antiperspirant stick compositions. Whilst this is function can be highly desirable in stick formulations, it can be less desirable in other formulations, such as those intended for roll-on application (*vide infra*).

WO 98/27954 (P&G) and WO 98/27948 (P&G) disclose antiperspirant gels and gel-solids optionally comprising 12-hydroxystearic acid in an anhydrous carrier fluid.

WO 04/071476 (Unilever) discloses suspension stick formulations optionally comprising 12-hydroxystearic acid; however, these formulations are again anhydrous.

12-hydroxystearic acid, amongst other compounds that are capable of reducing the melanin content of the skin, is known as skin lightening agent.

WO 09/153169 (Unilever) discloses cosmetic compositions comprising 12-hydroxystearic acid, amongst other agents, and the use of such compositions for lightening the skin. Since underarm darkening is a problem that has been observed by users of antiperspirant compositions, the incorporation of 12-hydroxystearic acid into such compositions is a particular object of the present invention.

The incorporation of 12-hydroxystearic acid specifically in oil-in-water emulsion compositions is challenging because of stability problems. In overcoming these problems, the inventors have also found a means of improving the appearance and condition of the skin. In certain embodiments of the invention, antiperspirancy is also delivered.

It is an object of the present invention to formulate stable oil-in-water emulsion cosmetic compositions comprising 12-hydroxystearic acid that are suitable for application to the surface of the human body from contact dispensers, in particular roll-on dispensers.

It is an object of certain embodiments of the present invention to provide means for delivering both antiperspirancy and skin appearance benefits from a single topically-applied composition.

According to a first aspect of the invention, there is provided an oil-in-water emulsion cosmetic composition comprising a continuous phase having water as the major constituent and dispersed therein droplets comprising a liquid oil, characterised in also comprising from 0.1 to 10% by weight of the total composition of independent solid particulates comprising 12-hydroxystearic acid having a particle size distribution such that 99% or more by weight have a particle size of less than 125 microns and a lamellar phase emulsifier system. According to a particular aspect of the invention, there is provided an oil-in-water emulsion antiperspirant composition comprising from 1 to 40% by weight of an aluminium and/or zirconium containing antiperspirant active and a continuous phase having water as the major constituent, characterised in also comprising from 0.1 to 10% by weight of the total composition of solid particulates comprising 12-hydroxystearic acid, 99% or more by weight of which have a particle size of less than 125 microns and a lamellar phase emulsifier system. According to a second aspect of the invention, there is provided a product for treating perspiration comprising an antiperspirant composition according to the particular aspect of the invention referred to in the paragraph immediately above and a roll-on applicator comprising a reservoir for holding the composition and a roll ball for applying the composition to the surface of the human body. Also disclosed herein is a method of improving the appearance of the skin comprising the topical application of a composition according to the first aspect of the invention.

The present invention overcomes the problems associated with incorporating 12-hydroxystearic acid into an oil-in-water emulsion composition, in particular an antiperspirant composition. Such compositions are difficult to formulate in a manner that gives long term stability, particularly at elevated temperatures, with thickening and separation being common problems.

The incorporation of 12-hydroxystearic acid into oil-in-water emulsion compositions presents particular problems because of the hydrophobic nature of this material, giving it affinity for the oil, and its ability to structure or thicken the emulsion which can lead to catastrophic rheological problems.

Underlying the problems addressed by the present invention is the thermodynamic instability of emulsions, a problem that is exacerbated by the presence of hydrophobic structuring agents such as 12-hydroxystearic acid. The difficulty of incorporating 12-hydroxystearic acid is particular severe in compositions comprising a lamellar phase emulsifier system, the 12-hydroxystearic acid seemingly disrupting the lamellar phase unless it is incorporated in the correct manner. The problem is also exacerbated by the presence of high levels of electrolytes, as found in antiperspirant compositions.

The instability problems referred to above can be a problem both on manufacture of emulsions and also during their storage. The present inventors have found particular stability problems for oil-in-water emulsions comprising 12-hydroxystearic acid when stored at elevated temperatures, as might be found during transport and warehouse storage in certain regions of the world.

Herein, amounts and concentrations of ingredients are percentages by weight of the total composition, unless otherwise indicated and ratios are ratios by weight.

Herein, the terms "oil" and "liquid oil" are used interchangeably and signify water-insoluble organic material that is liquid at 20°C. Any material having a solubility of less than 0.1g/100g at 20°C is considered to be insoluble.

Herein, references to viscosities should be understood to be viscosities measured at 25°C using standard methods.

A preferred oil for use in accordance with the present invention is a fragrance oil, sometimes alternatively called a perfume oil. The fragrance oil may comprise a single fragrance or component more commonly a plurality of fragrance components. Herein, fragrance oils impart an odour, preferably a pleasant odour, to the composition. Preferably, the fragrance oil imparts a pleasant odour to the surface of the human body the composition is applied to the same.

The amount of fragrance oil in the composition is commonly up to 3% advantageously is at least 0.5% and particularly from 0.8% to 2%.

The total amount of oil in the composition is preferably from 0.1 to 20%, more preferably from 0.5 to 10%, and most preferably at from 2 to 8% by weight of the total composition. In certain preferred embodiments, particularly those also comprising an aluminium and/or zirconium containing antiperspirant active, the oil is present at greater than 2.5% and less than 6% by weight of the total composition.

In certain embodiments, it is preferred to include an oil, other than a fragrance oil, that has a relatively low viscosity, by which is meant less 250 cS (mm².s⁻¹). Such oils can improve the sensory properties of the composition on application and can lead to other benefits such as emolliency. They can, however, accentuate the stability problems of the composition, making the method of incorporation of the 12-hydroxystearic acid even more critical.

Suitable oils can be selected from alkyl ether oils having a boiling point of above 100°C and especially above 150°C, including polyalkyleneglycol alkyl ethers. Such ethers desirably comprise between 10 and 20 ethylene glycol or propylene glycol units and the alkyl group commonly contains from 4 to 20 carbon atoms. The preferred ether oils include polypropylene glycol alkyl ethers such as PPG-14-butylether and PPG-15-stearyl ether.

Suitable oils can include one or more triglyceride oils. The triglyceride oils commonly comprise the alkyl residues of aliphatic C₇ to C₂₀ alcohols, the total number of carbon atoms being selected in conjunction with the extent of olefinic unsaturation and/or branching to enable the triglyceride to be liquid at 20°C. One example is jojoba oil. Particularly preferably, in the triglyceride oil the alkyl residues are linear C₁₈ groups having one, two or three olefinic degrees of unsaturation, two or three being optionally conjugated, many of which are extractable from plants (or their synthetic analogues), including triglycerides of oleic acid, linoleic acid, conjugated linoleic acids, linolenic acid, petroselenic acid, ricinoleic acid, linolenelaidic acid, trans 7-octadecenoic acid, parinaric acid, pinolenic acid, punicic acid, petroselenic acid and stearidonic acid.

Suitable oils can include those derived from unsaturated C₁₈ acids, including coriander seed oil, impatiens balsimina seed oil, parinarium laurinarium kernel fat oil, sabastiana brasilinensis seed oil, dehydrated castor seed oil, borage seed oil, evening primrose oil, aquilegia vulgaris oil, sunflower (seed) oil and safflower oil. Other suitable oils are obtainable from hemp, and maize corn oil. An especially preferred oil by virtue of its characteristics and availability comprises sunflower (seed) oil.

Further suitable oils, that can also be emollient oils, comprise alkyl or alkyl-aryl ester oils having a boiling point of above 150°C (and a melting point of below 20°C). Such ester oils include oils containing one or two alkyl groups of 12 to 24 carbon atoms length, including isopropyl myristate, isopropyl palmitate and myristyl palmitate. Other non-volatile ester oils include alkyl or aryl benzoates such C₁₂₋₁₅ alkyl benzoate, for example Finsolv TN™ or Finsolv Sun™.

A further class of suitable oils comprises non-volatile dimethicones, often comprising phenyl or diphenylene substitution, for example Dow Corning 200 350cps or Dow Corning 556.

The continuous phase of the emulsion is predominately comprised of water, that is to say, water is the major constituent of the continuous phase. The content of water in the continuous phase is preferably 60% or greater, more preferably 70% or greater, and most preferably 75% or greater. The content of water in the total composition is preferably 50% or greater, more preferably 60% or greater, and most preferably 65% or greater.

Other components that may be present in the continuous phase include short chain (C₂-C₄) alcohols and especially polyols such glycerol, ethylene glycol, propylene glycol and polymers thereof, in particular poly(ethylene glycol) and poly(propylene glycol). Poly(ethylene glycol) of average molecular weight 200 to 600 is a preferred component. Such components may add to the sensory properties of the composition and, when included, are typically present at from 0.5 to 10% of the total composition.

When employed, the antiperspirant active is one containing aluminium and/or zirconium, as commonly used in the art for this purpose. Such actives are watersoluble and are typically fully dissolved in the aqueous continuous phase of the composition.

The antiperspirant active is typically selected from astringent salts, including both inorganic salts, salts with organic anions, and complexes. Preferred antiperspirant actives are aluminium, zirconium, and aluminium-zirconium chlorides, oxychlorides, and chlorohydrates salts. Particularly preferred antiperspirant actives are polynuclear in nature, meaning that the cations of the salt are associated into groups comprising more than one metal ion.

Aluminium halohydrates are especially preferred antiperspirant actives and may be defined by the general formula Al₂(OH)ₓQ_{y}.wH₂0, in which Q represents chlorine, bromine or iodine, x is variable from 2 to 5 and x + y = 6 while wH₂O represents a variable amount of hydration. Aluminium chlorohydrate (ACH) is the most preferred active.

Zirconium salts are usually defined by the general formula ZrO(OH)₂₋ₓQₓ.wH₂O in which Q represents chlorine, bromine or iodine; x is from about 1 to 2; w is from about 1 to 7; and x and w may both have non-integer values. Particular zirconium salts are zirconyl oxyhalides, zirconiun hydroxyhalides, and combinations thereof.

Antiperspirant actives as used in the invention may be present as mixtures or complexes. Suitable aluminium-zirconium complexes often comprise a compound with a carboxylate group, for example an amino acid. Examples of suitable amino acids include tryptophan, β-phenylalanine, valine, methionine, β-alanine and, most preferably, glycine.

In some embodiments, it is desirable to employ complexes of a combination of aluminium halohydrates and zirconium chlorohydrates with amino acids such as glycine, which are disclosed in US 3,792,068 (Procter and Gamble Co.). Certain of these Al/Zr complexes are commonly called ZAG in the literature. ZAG actives generally contain aluminium, zirconium and chloride with an Al/Zr ratio in a range from 2 to 10, especially 2 to 6, an Al/Cl ratio from 2.1 to 0.9 and a variable amount of glycine.

Antiperspirant actives are preferably incorporated in an amount of from 0.5 to 60%, particularly from 5 to 30% or 40% and especially from 10% to 30% of the total composition.

The concentration of antiperspirant active in the aqueous continuous phase is preferably from 1% to 40%, more preferably from 5% to 30%, and most preferably from 10% to 20% by weight of the continuous phase. The emulsifier(s) used in compositions of the present invention form(s) a lamellar phase emulsifier system in the composition. Such systems may be readily identified by means of optical microscopy. Such systems lead to good emulsion stability, particularly in compositions comprising an aluminium and/or zirconium containing antiperspirant active; however, they are particularly sensitive to the presence of 12-hydroxystearic acid, necessitating its addition in the correct form (*vide supra*)*.*

In certain preferred embodiments, the composition comprises a non-ionic emulsifier system. Such an emulsifier system conveniently has a mean HLB value in the region of from about 5 to about 12 and particularly from 6 to about 10. In the preferred embodiments referred to in the paragraph immediately above, an especially desired mean HLB value is from 6 to 9. Such a mean HLB value can be provided by selecting an emulsifier having such an HLB value, or more preferably by employing a combination of at least two emulsifiers, a first (lower) HLB emulsifier having an HLB value in the range of from 2 to 6.5, such as in particular from 4 to 6 and a second (higher) HLB emulsifier having an HLB value in the range of from about 6.5 to 18 and especially from about 12 to about 18. When a combination of emulsifiers is employed, the average HLB value can be calculated as a weight average of the HLB values of the constituent emulsifiers. Lamellar phase emulsifier systems preferably comprise two non-ionic surfactants, optionally selected as suggested in the paragraph immediately above. In a particular embodiment a first emulsifier is a fatty alcohol, such as cetyl and/or stearyl alcohol and a second emulsifier is much more hydrophilic, having a HLB of from about 6.5 to 18 and especially from about 12 to about 18

An especially desirable range of emulsifiers comprises a hydrophilic moiety provided by a polyalkylene oxide (polyglycol), and a hydrophobic moiety provided by an aliphatic hydrocarbon, preferably containing at least 10 carbons and commonly linear. The hydrophobic and hydrophilic moieties can be linked via an ester or ether linkage, possibly via an intermediate polyol such as glycerol. A preferred range of emulsifiers comprises polyethylene glycol ethers.

Preferably the hydrophobic aliphatic substituent contains at least 12 carbons, and is derivable from lauryl, palmityl, cetyl, stearyl, and behenyl alcohol, and especially cetyl, stearyl or a mixture of cetyl and stearyl alcohols or from the corresponding carboxylic acids.

The polyalkylene oxide is often selected from polyethylene oxide and polypropylene oxide or a copolymer of ethylene oxide and especially comprises a polyethylene oxide. The number of alkylene oxide and especially of ethoxylate units within suitable emulsifiers is often selected within the range of from 2 to 100. Emulsifiers with a mean number of ethoxylate units in the region of 2 can provide a lower HLB value of below 6.5 and those having at least 4 such units provide a higher HLB value of above 6.5 and especially those containing at least 10 ethoxylate units which provide an HLB value of above 10. A preferred combination comprises a mixture of an ethoxylate containing 2 units and one containing from 10 to 40 units, such as from 15 to 30 or desirably from 20 to 25. Particularly conveniently, the combination of emulsifiers comprises steareth-2 and a selection from steareth-15 to steareth-30.

It is desirable to employ a mixture of ethoxylated alcohol emulsifiers in a weight ratio of emulsifier having a lower HLB value of less than 6.5 to emulsifier having a higher HLB value of greater than 8 of from 2:1 to 6:1 and particularly from 4:1 to 6:1.

The total proportion of emulsifiers in the composition is usually at least 1 % and particularly at least 2% by weight. Commonly, the emulsifiers are not present at above 10%, often not more than 7% by weight and in many preferred embodiments up to 6% by weight. An especially desirable concentration range for the emulsifiers is from 2.5 to 5% by weight.

The solid particulates comprising 12-hydroxystearic acid preferably comprise a high level of 12-hydroxystearic acid, by which is meant at least 50%, more preferably at least 75% and most preferably at least 80% by weight, ignoring any associated surfactant. The other components in the solid particulates are typically alternative solid fatty acids.

It is preferred that the solid particulates comprise only a low level of components which, in isolation, would be liquids at 20°C. It is preferred that the total level of such components is less than 10%, more preferably less than 5%, and most preferably less than 2% by weight. This is particularly true when the particulates are dry milled to give them their particle size distribution and especially when they are dry milled and have the preferred particle sizes indicated below.

The solid particulates comprising 12-hydroxystearic acid are present at from 0.1 to 10%, more preferably from 0.25 to 8%, and most preferably at from 0.5 to 5% by weight of the total composition. In certain preferred embodiments, particularly those also comprising an aluminium and/or zirconium containing antiperspirant active, the solid particulates comprising 12-hydroxystearic acid are present at greater than 1 % and less than 3% by weight of the total composition.

It is preferable that the solid particulates are dry milled. By this it is meant that the particulates are reduced in particle size by milling or grinding them in the absence on any additional fluid and at a temperature at which the particulates are solid. This is done prior to their incorporation into the emulsion composition.

Throughout this description, references to particle size should be understood to be to the maximum dimension of the particle or particulate, these latter terms being used interchangeably.

The particle size distribution of the solid particulates comprising 12-hydroxystearic acid is preferably such that at least 99% by weight of them have a particle size of less than 125 microns. The mean particle size of the particulates (by volume) is preferably less than 75 microns and more preferably less than 50 microns. Particle size distributions may be analysed using light scattering techniques on instruments such as the Malvern Mastersizer and/or the Sympatec Helos particle size analyser.

In certain embodiments, the particulates have a non-ionic surfactant of relative high HLB, by which is meant 10 or greater, associated with them. This can enhance the mixing of the solid particulates into the composition and can improve formulation stability, particularly when the particulates comprise the preferred high levels of 12-hydroxystearic acid indicated in the above paragraph. Typically, the non-ionic surfactant is associated with the surfaces of the particulates.

A preferred high HLB non-ionic surfactant for use in the above aspect of the invention is an alkoxylated fatty acid of fatty alcohol chain length C₁₆-C₁₈ and degree of alkoxylation from 10 to 30. Particularly preferred is Steareth 20, such as available under the trade name Brij S20 PA, from Croda Europe Ltd.

In certain alternative embodiments, the particulates have a powdered flow aid associated with them. In these embodiments, the particulates tend to be added as a dry powder and the powdered flow aid assists the flow of the powder into the formulation. A preferred flow aid is silica, in particular hydrophobically modified silica, such as silica dimethyl silylate, available as HDK® H30 from Wacker Chemie GmbH. When employed, such flow aids are typically used at from 1 to 30% by weight of the solid particulates.

An optional ingredient that may further ameliorate the emulsion instability is a particulate silica such as an amorphous silica, and especially a fumed silica.

Further optional ingredients include preservatives, colorants, and anti-microbials including conventional deodorant agents. Opacifying agents, such as titanium dioxide, may also be employed in some formulations.

Particularly preferred additional ingredients include other skin care actives, especially additional skin care actives that can enhance the lightening of the skin.

In certain embodiments, waxes are preferred additional ingredients. Waxes are solid, hydrophobic, organic compounds, typically having a melting point of greater than 30°C. They can serve to add to the rheological stability and thicken the composition. Certain waxes can form part of a lamellar phase emulsifier system (*vide supra*). Preferred waxes are fatty alcohols, particularly those having a chain length of from 16 to 18 carbon atoms, and fatty esters, particularly glyceryl esters and especially glyceryl mono-esters of fatty acids having from 16 to 18 carbon atoms.

When waxes are employed, the total amount is preferably from 1 to 30%, more preferably from 5 to 20, and most preferably from 8 to 15% by weight of total composition.

Particularly when the solid particulates comprising 12-hydroxystearic acid have a low content of liquid components, as described in the above paragraph, it is preferred that they are milled to give them the required or even preferred particle size as described above before the particulates are incorporated into the final composition. Preferably this is done by dry milling, that is to say, in the absence of any liquid.

In certain aspects of the invention, the solid particulates are added as a dispersion in water, preferably with a non-ionic emulsifier of relative high HLB, by which is meant 10 or greater. This enhances the mixing of the solid particulates into the composition and can improve formulation stability, particularly when the particulates comprise the preferred high levels of 12-hydroxystearic acid indicated in the above paragraph. The dispersion preferably has the high HLB non-ionic emulsifier present at a level of from 1 to 20% by weight of the total weight of the particulates and more preferably at a level of from 2 to 15% by weight thereof.

The concentration of particulates in the aqueous dispersion is preferably from 5 to 60% by weight and more preferably from 10 to 50% by weight.

A preferred high HLB non-ionic emulsifier for use in the above aspect of the invention is an alkoxylated fatty acid of fatty alcohol chain length C₁₆-C₁₈ and degree of alkoxylation from 10 to 30. Particularly preferred is Steareth 20, such as available under the trade name Brij S20 PA, from Croda Europe Ltd.

In preferred embodiments, the solid particulates are added as a dry powder, preferably with a flow aid, such as silica, preferred options for which are described earlier.

Further background on the method of preparation of gel particulates described in the above paragraph may be found in articles by Kilrov et al in Physicochemical and Engineering Aspects, 328, 1-7, 2008 and J. Phys. Chem. B, 113, 11101-11108,2009.

In certain preferred aspect of the invention, the dry particulates are added sub-surface, that is to say, at a point under the upper surface of the liquid composition being prepared. This is preferably done by having the composition at a pressure of less than atmospheric and opening a sub-surface valve to allow the ingress of the particulates. This method is particularly suitable when the particulates are added as a dry powder and especially so when the dry powder is added with a flow aid, such as silica.

The compositions used in the present invention are very suitable for dispensing via a roll-on dispenser, for example any upright dispenser such as described in EP1175165 or an invert dispenser such as described in US6511243 or WO05/007377. Invert indicates that the dispenser stands stably with its dispensing ball below the formulation reservoir.

The invention formulation is applied by rolling the ball of the dispenser topically across the skin surface, depositing a film of fluid on the skin. Commonly the dispenser is wiped across the skin between 4 and 10 strokes, depending on the habit of the user and the diameter of the ball. Commonly from 0.2 to 0.5g fluid is deposited in each armpit per application.

Having given a brief summary of the present invention and preferred embodiments, a more detailed description of certain embodiments will be given by way of example only.

### Examples

Examples according to the invention are indicated by number and comparative examples are indicated by letter. All amounts and ratios are by weight and tabulated amounts are percentages, unless otherwise indicated.

The method of preparation of Comparative Example A and Examples 1 and 2 involved the following steps. The water, aluminium chlorohydrate, and glycerol were placed in a vessel equipped with a circulation loop and heated to 40-45°C with agitation *via* circulation through the loop and via a mixer in the vessel. The Steareth 20 was added and allowed to fully dissolve into the mixture (but approximately a third added with the 12-HSA in Example 2, *vide infra*). In a separate vessel, the Steareth 2 was melted into the sunflower oil by heating to approximately 65°C. This oil phase was injected into the aqueous phase (water, aluminium chlorohydrate, glycerol, and Steareth 20) *via* the circulation loop and the resulting mixture subjected to high shear mixing (8000 rpm) using an in-line dynamic mixer (Ultra-Turrax T50). The mixture was cooled to 40°C and the fragrance added with stirring. Finally, the mixture was subjected to high shear mixing (8000 rpm) again using the in-line dynamic mixer (Ultra-Turrax T50).

In the preparation of Comparative Example A, the 12-hydroxystearic acid was melted into the oil phase and the resulting oil phase injected into the aqueous phase as described in the paragraph above. All other processing steps were as described in the paragraph above.

Comparative Example A exhibited poor stability when stored at 45°C, a separate layer appearing within one week.

**Table 1**

| **Example** | **A** | **1** | **2** | **3** | **4** |
|---|---|---|---|---|---|
| Component: | | | | | |
| 12-hydroxystearic acid: | | | | | |
| Melted into oil phase | 2 | | | | |
| Added as powder | | 2 | 2 | | |
| Added as gel particulates | | | | 2 | 2 |
| Sunflower seed oil (1) | 4.0 | 4.0 | 4.0 | 4.0 | 4.0 |
| Steareth 2 (2) | 2.3 | 2.3 | 2.3 | 2.3 | 2.3 |
| Steareth 20 (3) | 0.9 | 0.9 | 0.9 | 0.9 | 0.9 |
| Aluminium chlorohydrate (4) | 15.0 | 15.0 | 15.0 | 15.0 | 15.0 |
| Glycerol | 4.0 | 4.0 | 4.0 | 4.0 | 4.0 |
| Fragrance | 1.0 | 1.0 | 1.0 | 1.0 | 1.0 |
| Water | To 100 | To 100 | To 100 | To 100 | To 100 |

| | | | | | |
|---|---|---|---|---|---|
| (1) Akosun, ex Karlshams. (2) Brij S2 PA, ex Croda. (3) Brij S20 PA, ex Croda. (4) Added as a 50% aqueous solution, ACH-50, ex Reheis. | | | | | |

In the preparation of Example 1, the 12-hydroxystearic acid was added as a dry powder immediately after the fragrance addition. The 12-hydroxystearic acid had a particle size reduced by drying milling such that 99% *by weight* was 125 micron or less and the mean particle size (by volume) was 32 microns, measured using a Sympatec Helos particle size analyser. The mixture was stirred to achieve a homogeneous composition.

Example 1 exhibited reasonable stability when stored at 45°C, being stable for one week.

In the preparation of Example 2, the 12-hydroxystearic acid was added as a suspension in water, wetted by Steareth 20, immediately after the fragrance addition. The 12-hydroxystearic acid used was the same as for Example 1, having a particle size reduced to less than 125 micron by dry milling and a volume mean distribution of 32 microns. The suspension contained 11.56% 12-hydroxystearic acid; 1.73% Steareth 20 and 86.71% water and was added with stirring to the main composition at a weight ratio of approximately 17:83 to give the indicated total composition.

Example 2 exhibited good stability when stored at 45°C, being stable for over 4 weeks.

### Example 3

12-hydroxystearic acid and sunflower seed oil were heated together to approximately 80°C. The resulting molten oil phase was added to water and Steareth 20 at 80°C with stirring. The resulting crude emulsion (3.33% Steareth 20; 13.33% sunflower seed oil; 6.67% 12-hydroxystearic acid; and water to 100%) was sonicated at 80°C at high power using a Soniprobe type 7532B (ex Dow Instruments, Ltd.) and then crash cooled by adding cold water (at less than 10°C) at a weight ratio of 40:60 to give a suspension of gel particulates having a composition of 2.0% Steareth 20; 8.0% sunflower seed oil; 4.0% 12-hydroxystearic acid; and 86.0% water.

The particle size distribution of the gel particle suspension was analysed using light scattering techniques on a Malvern Mastersizer and a D[4,3] value of 0.13 microns recorded.

Water, aluminium chlorohydrate, and glycerol were placed in a vessel equipped with a circulation loop and heated to 50-55°C with agitation *via* circulation through the loop. Steareth 20 was added and allowed to fully dissolve into the mixture. In a side pot, Steareth 2 was melted by heating to about 65°C. The molten Steareth 2 was then injected into the aqueous phase and the mixture subjected to high shear mixing (8000 rpm) using an in-line dynamic mixer (Ultra-Turrax T50). A lamellar dispersion resulted, having the composition 30.6% aluminium chlorohydrate; 0.6% Steareth 20; 8.2% glycerol; 4.7% Steareth 2 and water to 100%.

The suspension of gel particles, at ambient temperature, was added to the lamellar dispersion, at 40°C, with stirring, followed by the fragrance, also with stirring. The weight ratio for this mixing step was 50:49:1 respectively, giving the final composition indicated in Table 1.

Example 3 exhibited reasonable stability when stored at 45°C, being stable for two weeks.

### Example 4

12-hydroxystearic acid was melted by heating to approximately 80°C. The resulting melt was added to water and Steareth 20 at 80°C with stirring. The resulting crude emulsion (3.33% Steareth 20; 6.67% 12-hydroxystearic acid; and water to 100%) was sonicated at 80°C at high power using a Soniprobe type 7532B (*ex* Dow Instruments, Ltd.) and then crash cooled by adding cold water (at less than 10°C) at a weight ratio of 40:60 to give a suspension of gel particulates

having a composition of 2.0% Steareth 20; 4.0% 12-hydroxystearic acid; and water to 100%.

The particle size distribution of the gel particle suspension was analysed using light scattering techniques on a Malvern Mastersizer and a D[4,3] value of 3.1 microns recorded.

Water, aluminium chlorohydrate, and glycerol were placed in a vessel equipped with a circulation loop and heated to 50-55°C with agitation *via* circulation through the loop. Steareth 20 was added and allowed to fully dissolve into the mixture. In a side pot, Steareth 2 was melted into sunflower seed oil by heating to about 65°C. The hot mixture of Steareth 2 and sunflower seed oil was then injected into the aqueous phase and the mixture subjected to high shear mixing (8000 rpm) using an in-line dynamic mixer (Ultra-Turrax T50). The resulting emulsion had the composition 30.6% aluminium chlorohydrate; 0.6% Steareth 20; 8.2% glycerol; 4.7% Steareth 2; 8.2% sunflower seed oil and water to 100%.

The suspension of gel particles, at ambient temperature, was added to the emulsion composition, at 40°C, with stirring, followed by the fragrance, also with stirring. The weight ratio for this mixing step was 50:49:1 respectively, giving the final composition indicated in Table 1.

Example 4 exhibited reasonable stability when stored at 45°C, being stable for two weeks.

Comparative Example B of Table 2 was made in a manner analogous to Comparative Example A. Like Comparative Example B, it exhibited poor rheological characteristics and was unacceptably thick.

Example 5 of Table 2 was prepared in a manner similar to Example 2, although the late-added suspension of 12-hydroxystearic acid in water (4.2% by weight) did not have any surfactant present. The 12-hydroxystearic acid suspension also had the niacinamide dissolved in it and was added at a temperature of 50°C. In this example, the sunflower seed oil was added after the 12-hydroxystearic acid suspension at a temperature of 40°C.

Example 5 was found to have satisfactory rheological properties and was not unacceptably thick.

**Table 2**

| **Example** | | **B** | **5** |
|---|---|---|---|
| Component: | Further details: | | |
| 12-hydroxystearic acid: | As Table 1 | | |
| Melted into oil phase | | 1.0 | |
| Added as powder | | | 1.0 |
| Glyceryl stearate | Cutina GMS-V ex Cognis | 7.5 | 7.5 |
| Cetearyl alcohol and PEG-20 stearate (4:1) | Polawax GP200 ex Croda | 5.0 | 5.0 |
| Sunflower seed oil | As Table 1 | 4.0 | 4.0 |
| Titanium dioxide | Opacifier | 0.2 | 0.2 |
| Niacinamide | | 0.1 | 0.1 |
| White mineral oil | Blandol ex Sonneborn | 1.0 | 1.0 |
| Cetearyl alcohol | Lanette O ex Cognis | 1.0 | 1.0 |
| Glycerol | | 4.0 | 4.0 |
| Water and minors | Including low levels of preservatives | To 100 | To 100 |

Examples 6 to 10 in Table 3 and Examples 11 to 13 in Table 4 were prepared by the same method as described for Example 2 (addition of 12-hydroxystearic acid suspension wetted by Steareth 20). Example 14 in table 4 was prepared by the same method as described for Example 1 (addition of 12-hydroxystearic acid as a powder).

Examples 6 to 8 exhibited good stability when stored at 45°C, being stable for 4 weeks. Examples 9 and 14 exhibited excellent stability when stored at 45°C, being stable for over 12 weeks.

**Table 3**

| **Example** | **6** | **7** | **8** | **9** | **10** |
|---|---|---|---|---|---|
| Component: | | | | | |
| 12-hydroxystearic acid | 2 | 2 | 2 | 2 | 2 |
| Sunflower seed oil (1) | 4.0 | 4.0 | 4.0 | 4.0 | 4.0 |
| Steareth 2 (2) | 3.25 | 3.5 | 3.5 | 3.75 | 4.0 |
| Steareth 20 (3) | 0.9 | 0.6 | 1.37 | 0.9 | 0.9 |
| Aluminium chlorohydrate (4) | 15.0 | 15.0 | 15.0 | 15.0 | 15.0 |
| Glycerol | 4.0 | 4.0 | 4.0 | 4.0 | 4.0 |
| Fragrance | 1.0 | 1.0 | 1.0 | 1.0 | 1.0 |
| Water | To 100 | To 100 | To 100 | To 100 | To 100 |

**Table 4**

| **Example** | **11** | **12** | **13** | **14** |
|---|---|---|---|---|
| Component: | | | | |
| 12-hydroxystearic acid | 1.0 | 0.5 | 0.5 | 2 |
| Sunflower seed oil (1) | 4.0 | 4.0 | 4.0 | 4.0 |
| Steareth 2 (2) | 3.18 | 2.89 | 3.18 | 3.75 |
| Steareth 20 (3) | 0.9 | 0.9 | 0.9 | 0.9 |
| Aluminium chlorohydrate (4) | 15.0 | 15.0 | 15.0 | 15.0 |
| Glycerol | 4.0 | 4.0 | 4.0 | 4.0 |
| Fragrance | 1.0 | 1.0 | 1.0 | 1.0 |
| Water | To 100 | To 100 | To 100 | To 100 |

## Claims

1. An oil-in-water emulsion cosmetic composition comprising a continuous phase having water as the major constituent and dispersed therein droplets comprising a liquid oil, **characterised in** also comprising from 0.1 to 10% by weight of the total composition of independent solid particulates comprising 12-hydroxystearic acid having a particle size distribution such that 99% or more by weight have a particle size of less than 125 microns and a lamellar phase emulsifier system.

2. An emulsion cosmetic composition according to claim 1, comprising from 1 to 40% by weight of an aluminium and/or zirconium containing antiperspirant active dissolved in the continuous phase.

3. An emulsion cosmetic composition according to claim 1 or 2, comprising a non-ionic emulsifier system having a weighted average HLB of from 5 to 12.

4. An emulsion cosmetic composition according to any preceding claims, comprising a liquid oil, other than a fragrance oil, having a viscosity of less than 250 mm²s⁻¹.

5. An emulsion cosmetic composition according to any preceding claims, comprising dry milled 12-hydroxystearic acid.

6. An emulsion cosmetic composition according to any preceding claims, wherein the solid particulates comprise less than 10% by weight of components which, in isolation, would be liquids at 20°C.

7. A product for treating perspiration comprising a composition according to any of claims 2 to 6 and a roll-on applicator comprising a reservoir for holding the composition and a roll ball for applying the composition to the surface of the human body.

## Patentansprüche

1. Kosmetische ÖI-in-Wasser-Emulsionszusammensetzung, die eine kontinuierliche Phase mit Wasser als Hauptbestandteil und darin dispergierte Tröpfchen, die ein flüssiges Öl umfassen, aufweist, **dadurch gekennzeichnet, dass** sie ferner 0,1 bis 10 Gewichts-% der gesamten Zusammensetzung unabhängige feste Partikel, die 12-Hydroxystearinsäure umfassen, mit einer Partikelgrößenverteilung, so dass 99 Gewichts-% oder mehr eine Partikelgröße von weniger als 125 Mikrometer aufweisen, und ein Emulgatorsystem mit lamellarer Phase umfasst.

2. Kosmetische Emulsionszusammensetzung nach Anspruch 1, die 1 bis 40 Gewichts-% eines Aluminium und/oder Zirkonium enthaltenden schweißhemmenden Wirkstoffs, der in der kontinuierlichen Phase gelöst ist, umfasst.

3. Kosmetische Emulsionszusammensetzung nach Anspruch 1 oder 2, die ein nicht-ionisches Emulgatorsystem mit einem gewichteten durchschnittlichen HLB von 5 bis 12 umfasst.

4. Kosmetische Emulsionszusammensetzung nach irgendeinem vorhergehenden Anspruch, das ein flüssiges Öl, anders als ein Duftstofföl, mit einer Viskosität von weniger als 250 mm²s⁻¹ umfasst.

5. Kosmetische Emulsionszusammensetzung nach irgendeinem vorhergehenden Anspruch, das trocken vermahlene 12-Hydroxystearinsäure umfasst.

6. Kosmetische Emulsionszusammensetzung nach irgendeinem vorhergehenden Anspruch, wobei die festen Partikel weniger als 10 Gewichts-% Bestandteile umfassen, welche, in Isolierung, bei 20°C Flüssigkeiten wären.

7. Produkt zur Behandlung von Schweißbildung, umfassend eine Zusammensetzung nach irgendeinem der Ansprüche 2 bis 6 und ein Rollenauftragsgerät, das ein Reservoir zur Aufnahme der Zusammensetzung und eine Rollkugel zum Auftragen der Zusammensetzung auf die Oberfläche des menschlichen Körpers umfasst.

## Revendications

1. Composition cosmétique d'émulsion huile-dans-eau comprenant une phase continue ayant de l'eau comme le constituant principal et dispersées dans celle-ci des gouttelettes comprenant une huile liquide, **caractérisée en ce qu'**elle comprend également de 0,1 à 10 % en masse de la composition totale de matières particulaires solides indépendantes comprenant de l'acide 12-hydroxystéarique ayant une distribution de taille de particule telle que 99 % ou plus en masse présentent une taille de particule inférieure à 125 microns, et un système d'émulsionnant en phase lamellaire.

2. Composition cosmétique d'émulsion selon la revendication 1, comprenant de 1 à 40 % en masse d'un actif d'antiperspirant contenant de l'aluminium et/ou du zirconium dissous dans la phase continue.

3. Composition cosmétique d'émulsion selon la revendication 1 ou 2, comprenant un système d'émulsionnant non-ionique ayant un HLB moyen pondéré de 5 à 12.

4. Composition cosmétique d'émulsion selon l'une quelconque des revendications précédentes comprenant une huile liquide, différente d'une huile de parfum, ayant une viscosité inférieure à 250 mm²s⁻¹.

5. Composition cosmétique d'émulsion selon l'une quelconque des revendications précédentes, comprenant de l'acide 12-hydroxystéarique broyé sec.

6. Composition cosmétique d'émulsion selon l'une quelconque des revendications précédentes, dans laquelle les matières particulaires solides comprennent moins de 10 % en masse de constituants qui, lors de l'isolement, seraient liquides à 20°C.

7. Produit de traitement de la transpiration comprenant une composition selon l'une quelconque des revendications 2 à 6 et un applicateur à rouleau comprenant un réservoir pour contenir la composition et une bille de rouleau pour appliquer la composition à la surface du corps humain.
